(19) Europäisches Patentamt
European Patent Office
Office européen des brevets

(11) EP 3 583 103 B1

(12) EUROPEAN PATENT SPECIFICATION

(45) Date of publication and mention of the grant of the patent:
**12.01.2022 Bulletin 2022/02**

(21) Application number: **18707229.3**

(22) Date of filing: **19.02.2018**

(51) Int Cl.:
***C07D 311/94*** ^(2006.01) ***C07C 59/42*** ^(2006.01)
***C07C 69/738*** ^(2006.01) ***C07C 69/608*** ^(2006.01)
***A61K 31/352*** ^(2006.01) ***A61K 31/201*** ^(2006.01)
***A61K 31/215*** ^(2006.01) ***A61P 37/06*** ^(2006.01)

(86) International application number:
**PCT/BR2018/050037**

(87) International publication number:
**WO 2018/148816 (23.08.2018 Gazette 2018/34)**

(54) **PLANT EXTRACTS ENRICHED WITH IPOLAMIIDE DERIVATIVES AS IMMUNOSUPPRESSANTS FOR TREATING IMMUNOLOGICAL DISORDERS**

MIT IPOLAMIDDERIVATEN ANGEREICHERTE PFLANZENEXTRAKTE ALS IMMUNSUPPRESSIVA ZUR BEHANDLUNG VON IMMUNOLOGISCHEN STÖRUNGEN

EXTRAITS DE PLANTES ENRICHIS AVEC DÉRIVÉS D'IPOLAMIDE EN TANT QU'IMMUNOSUPPRESSEURS POUR LE TRAITEMENT DE TROUBLES IMMUNOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2017 BR 102017003318**

(43) Date of publication of application:
**25.12.2019 Bulletin 2019/52**

(73) Proprietor: **Aché Laboratórios Farmacêuticos S.A.**
**07034-904 Guarulhos - SP (BR)**

(72) Inventors:
- **GUIMARÃES, Cristiano Ruch Werneck**
  **04087-002 São Paulo SP (BR)**
- **VITOR, Carlos Eduardo**
  **03111-010 São Paulo SP (BR)**
- **PESSA, Lisandra Ravanelli**
  **04118-095 São Paulo SP (BR)**
- **REIS, Romulo Dragani**
  **07072-080 Guarulhos SP (BR)**
- **MASCARELLO, Alessandra**
  **São Paulo, SP 05049-000 (BR)**
- **GAMA, Fernando Henrique De Souza**
  **07197-130 Guarulhos SP (BR)**

(74) Representative: **Brazzini, Silvia et al**
**Società Italiana Brevetti S.p.A.**
**Corso dei Tintori, 25**
**50122 Firenze (IT)**

(56) References cited:
**EP-A1- 0 045 837** **EP-A1- 1 145 709**
**EP-A1- 1 371 372** **WO-A1-03/094946**
**WO-A1-2008/098325** **WO-A1-2016/128471**
**CN-A- 103 120 699** **CN-A- 103 690 551**
**DE-A1- 19 644 422** **US-A- 3 703 543**

- **ADEBAJO, A. C. ET AL: "Hypoglycaemic constituents of Stachytarpheta cayennensis leaf", PLANTA MEDICA, vol. 73, no. 3, 22 February 2007 (2007-02-22), pages 241-250, XP002780026, ISSN: 0032-0943, DOI: 10.1055/s-2007-967125**
- **BIANCO, A. ET AL: "Acid-catalyzed rearrangements of iridoid aglycones. I. Behavior of lamiidol, a nonnatural lamiide derivative", TETRAHEDRON LETTERS, vol. 48, 1978, pages 4829-4832, XP002780027, ISSN: 0040-4039, DOI: 10.1016/S0040-4039(01)85744-0**
- **KHOBRAKOVA, V. B. ET AL: "Immunomodulating Activity of Extract of Gentiana Algida Pall", PHARMACEUTICAL CHEMISTRY JOURNAL, vol. 51, no. 5, August 2017 (2017-08), pages 384-387, XP002780028, ISSN: 0091-150X, DOI: 10.1007/s11094-017-1618-z**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 583 103 B1

- HOKI, KUNIHITO ET AL: "Chiral Molecular Motors Ignited by Femtosecond Pump-Dump Laser Pulses", JOURNAL OF PHYSICAL CHEMISTRY B, vol. 108, no. 15, 2004, pages 4916-4921, XP002782769, ISSN: 1520-6106, DOI: 10.1021/jp036437l
- KUNIHITO HOKI ET AL: "Mechanism of unidirectional motions of chiral molecular motors driven by linearly polarized pulses", JOURNAL OF CHEMICAL PHYSICS, vol. 119, no. 23, 15 December 2003 (2003-12-15), pages 12393-12398, XP055488425, US ISSN: 0021-9606, DOI: 10.1063/1.1621622
- SCHLOEGL, K.; FRIED, M.: "Ferrocenasymmetrie, 1. Mitt.: Darstellung und Racematspaltung von asymmetrischen Aminen und Aldehyden der Ferrocenreihe", MONATSHEFTE FUER CHEMIE, vol. 95, no. 2, 1964, pages 558-575, XP002782770, ISSN: 0026-9247
- BENKESER, ROBERT A.; BACH, JOSEPH L.: "Factors governing orientation in metalation reactions. III. Metalation of alkylferrocenes", JOURNAL OF THE AMERICAN CHEMICAL SOCIETY, vol. 86, no. 5, 1964, pages 890-895, XP002782771, ISSN: 0002-7863, DOI: 10.1021/ja01059a029
- ORHAN, ILKAY ERDOGAN ET AL: "Immunomodulatory properties of various natural compounds and essential oils through modulation of human cellular immune response", INDUSTRIAL CROPS AND PRODUCTS, vol. 81, 2016, pages 117-122, XP002782772, ISSN: 0926-6690, DOI: 10.1016/j.indcrop.2015.11.088
- KOBAYASHI, YUKO ET AL: "Inhibitory effects of geranium essential oil and its major component, citronellol, on degranulation and cytokine production by mast cells", BIOSCIENCE, BIOTECHNOLOGY AND BIOCHEMISTRY, vol. 80, no. 6, 2016, pages 1172-1178, XP002782773, ISSN: 1347-6947, DOI: 10.1080/09168451.2016.1148573
- LEE, JE-HYUK ET AL: "Anti-inflammatory activities of Chopi (Zanthoxylum piperitum A.P. DC) essential oil: Suppression of the inducible nitric oxide synthase and cellular adhesion", FOOD SCIENCE AND BIOTECHNOLOGY, vol. 18, no. 6, 2009, pages 1371-1378, XP002782774, ISSN: 1226-7708
- LEE, JE-HYUK ET AL: "Composition and anti-inflammatory activities of Zanthoxylum schinifolium essential oil: suppression of inducible nitric oxide synthase, cyclooxygenase-2, cytokines and cellular adhesion", JOURNAL OF THE SCIENCE OF FOOD AND AGRICULTURE, vol. 89, no. 10, 2009, pages 1762-1769, XP002782775, ISSN: 0022-5142, DOI: 10.1002/jsfa.3653
- YU, XIA ET AL: "A new linear monoterpene from the Chinese mangrove plant Cerbera manghas L", JOURNAL OF CHINESE PHARMACEUTICAL SCIENCES, vol. 18, no. 3, 2009, pages 232-235, XP009506549, ISSN: 1003-1057
- ELMASRI, WAEL A. ET AL: "Iridoid glycoside permethylation enhances chromatographic separation and chemical ionization", RAPID COMMUNICATIONS IN MASS SPECTROMETRY, vol. 30, no. 18, 2016, pages 2033-2042, XP002782776, ISSN: 0951-4198, DOI: 10.1002/rcm.7681
- BADEN, CHRISTIAN ULRICH ET AL: "Differing patterns of sequestration of iridoid glycosides in the Mecininae (Coleoptera, Curculionidae)", CHEMOECOLOGY, vol. 22, no. 2, 2012, pages 113-118, XP002782777, ISSN: 0937-7409, DOI: 10.1007/s00049-012-0103-0
- AKKOL, ESRA KUPELI ET AL: "Antinociceptive and anti-inflammatory activities of some Linaria species from Turkey", PHARMACEUTICAL BIOLOGY, vol. 47, no. 3, 2009, pages 188-194, XP002782778, ISSN: 1388-0209, DOI: 10.1080/13880200802465165
- VICCINI ET AL: "Ipolamiide and fulvoipolamiide from Stachytarpheta glabra (Verbenaceae): A structural and spectroscopic characterization", JOURNAL OF MOLECULAR STRUCTURE, ELSEVIER, AMSTERDAM, NL, vol. 875, no. 1-3, 1 March 2008 (2008-03-01), pages 27-31, XP022508309, ISSN: 0022-2860, DOI: 10.1016/J.MOLSTRUC.2007.03.056
- LEITAO, GILDA GUIMARAES ET AL: "Step-gradient CCC separation of phenylpropanoid and iridoid glycosides from roots of Stachytarpheta cayennensis", JOURNAL OF LIQUID CHROMATOGRAPHY & RELATED TECHNOLOGIES, vol. 28, no. 12-13, 2005, pages 2053-2060, XP002782779, ISSN: 1082-6076, DOI: 10.1081/JLC-200063672
- SCHAPOVAL, ELFRIDES E. S.: "Antiinflammatory and antinociceptive activities of extracts and isolated compounds from Stachytarpheta cayennensis", JOURNAL OF ETHNOPHARMACOLOGY, vol. 60, no. 1, 1998, pages 53-59, XP002782780, ISSN: 0378-8741, DOI: 10.1016/S0378-8741(97)00136-0

## Description

Field of the invention

The following invention provides isolated compounds and extracts with immunosuppressive activity and a process of producing extracts from plants of the genus *Stachytarpheta.* In this way, the present invention also provides compositions and their use for the treatment of immunological disorders. The present invention is in the fields of pharmacy, medicine and chemistry.

Background of the invention

Immunological disorders

Immunological disorders can be considered as any imbalance or malfunction of the immune system. This system is primarily responsible for assisting in the defense of the body against external or unknown agents through antibodies that recognize and fight harmful antigens.

Autoimmune diseases are an example of immunological disorders in which the organism starts to produce antibodies against their own molecules, making no distinction between endogenous and exogenous agents. In such cases, medicaments with immunosuppressive activity would be highly demanded to help patients relieve the symptoms caused by this type of disorder.

Autoimmune diseases, except for rheumatoid arthritis and autoimmune thyroiditis, are individually rare, but together they affect approximately 5% of the population of the western countries. Their etiology is not fully understood. In organspecific and systemic autoimmune diseases, it is observed a loss of the capacity of the immune system to distinguish what is self from what is not self. This ability, called self-tolerance, is maintained in the immunocompetent B and T cells by both central and peripheral mechanisms. The loss of self-tolerance may have intrinsic or extrinsic causes. Environmental factors such as bacterial and viral infections, exposure to physical and chemical agents such as UV, pesticides and drugs are examples of extrinsic causes. Intrinsic causes, that is, related to characteristics of the individual itself, are usually associated with polymorphisms of histocompatibility molecules, components of innate immunity, such as the complement system and *Toll-like* receptors, components of acquired immunity as regulatory lymphocytes and cytokines, in addition to hormonal factors which are under genetic control.

The therapeutic strategy in autoimmune diseases mainly consists in suppressing the immunological system using immunosuppressants, which act on the inhibition of the early stages of development of immunity. This therapy does not perform a selective immunosuppression, which led to the development of a variety of antibodies. Biological agents may be employed to inhibit the effect of cytokines, as occurs with anti-cytokine monoclonal antibodies or the use of soluble receptors that bind to the cytokine and block their effects on target cells. The cytokines are also used in biological therapy through analogous recombinant proteins that mimic the effect of the original cytokine. The main therapeutic targets in anti-cytokines therapy are the pro-inflammatory cytokines interleukin-1 (IL-1), TNF$\alpha$ and IL-6, and the main cytokine agonist therapy is performed with the use of type I interferons (IFN). In the type I interferon family, recombinant proteins of IFN$\alpha$ and of IFN$\beta$ are used in clinical practice, primarily for the treatment of viral hepatitis (hepatitis virus B and C) and of multiple sclerosis, respectively. For the latter, the proposed mechanism of action is the antagonism that the INF$\beta$ exerts against IFN$\gamma$, which has great importance in the physiopathology of multiple sclerosis. The IFN$\alpha$ has also been used in the treatment of muco-cutaneous, ocular and neurological manifestations of Behçet's disease and Churg-Strauss syndrome. Fontolizumab is a humanized anti-IFNy agent, which has been evaluated in patients with abnormalities in dendritic cells with good results.

Monoclonal antibodies have advantages in the treatment of autoimmune diseases when compared with conventional therapies as they are a targeted therapy, with specificity and high selectivity. However, they are used as a second line of treatment when there is no effectiveness in the control of autoimmune diseases with conventional therapies. In addition to restrictions on the efficacy, monoclonal antibodies still have several limitations related to cost and accessibility. Several autoimmune diseases still lack effective treatments, with good tolerability by patients.

Natural products have been used for centuries in the treatment of different diseases. Recently, major efforts have been made in the development of new research of herbal products with immunosuppressive effects. For example, several clinical trials performed in the United States have already shown significant benefits of *T. wilfordii* extract in patients with rheumatoid arthritis. Although there are several species and their active constituents with mechanisms of action described, there is still a vast field to be explored regarding *in vitro* and *in vivo* investigations and future clinical trials in immunologically based diseases. These natural products should be formulated in appropriate pharmaceutical compositions in order to ensure the effectiveness of the treatment of autoimmune diseases.

An autoimmune disease for which, so far, there is no effective medication, much less a medication option obtained from natural extracts, can be exemplified by vitiligo. Vitiligo is commonly associated with loss of functional

melanocytes and is considered the most common acquired depigmentation disorder in humans, affecting at least 0.5% of the world population. It is characterized by the development of white maculae, resulting from the loss of epidermal melanocytes, which can result in cellular destruction through a specific cytotoxic immune response to melanocytes and in damage to the adhesion system thereof.

Multiple mechanisms have been associated with vitiligo such as genetic predisposition, environmental activations, metabolic anomalies and changes in the immune and inflammatory responses. In addition, conditions such as exposure to ultraviolet radiation and oxidative stress are known to aggravate this condition.

Due to the lack of specific knowledge about the initial onset of the disease, several studies try to elucidate the biological pathways involved in this pathogenesis. Most of them indicate the complexity and the challenges related to such disease, being very difficult to find an efficient treatment. We must not forget that most immunological disorders have an important social impact, causing a high level of psychological stress for the patients. To date, there is no intervention capable of delivering the cure of vitiligo.

In this way, it becomes eminent the need to identify new compounds capable of promoting immunosuppressive activity with long-term effect. Thus, the present invention addresses this gap in the treatment of immunological disorders through compounds which, when isolated, demonstrate immunosuppressive activity and active extracts comprising groups of active compounds, which are obtained in a manner as follows.

Highlighting the complexity and lack of scientific knowledge about vitiligo, it was believed so far that the structure of ipolamiide in its intact form could be associated with immunosuppressive activity, relevant to the treatment of patients with vitiligo. However, in the present invention we demonstrate that this activity results from specific derivatives of ipolamiide and of the extracts comprising such compounds, preferably obtained by the processes of production described herein. Both compounds and extracts present immunosuppressive activity confirmed experimentally. This activity is demonstrated herein by blocking the activation of CD8+ T cells and reduction of IFN-$\gamma$ secretion. This mechanism has been shown to be promising for vitiligo in view of recent clinical findings related to the quantification of these components in the skin of patients. Specifically, high concentrations of CD8+ T cells and IFN-$\gamma$ are linked to the apoptosis of melanocytes and, therefore, the modulation of these is a promising mechanism of action.

WO 2016/128471 A1 discloses an immunologically active plant mixture comprising at least on eplant extract selected from the Asteraceae family, the Verbenaceae family, and/or the Burseraceae family.

WO 2008/098325 A1 discloses a process to obtain a compound and a standard pharmaceutical product from one or more parts of plants of the Stachytarpheta (Verbenaceae family) species.

WO 03/094946 A1 discloses a method for treating and/or preventing hepatic disease conditions in a subject mammals including human beings, by administration of a composition comprising 2'-p-hydroxy-benzoyl-massaenosidic acid from plant Vitex negundo.

CN 103690551 A discloses iridoid glycoside extracts in cleavers, an extraction method and medical applications thereof in aspects of anti-inflammation and nerve protection.

Adebajo A.C. et al., in Planta Medica 2007, vol. 73, no. 3, 241-250 disclose the use of extracts from *Stachytarpheta cayennensis* leaves for treating diabetes.

CN 103120699 A discloses iridoid compounds for preparing medicines for treating or preventing SARS.

EP 1145709 A1 discloses the use of plants extracts of the Solanaceae family or the Physalis, Stachytarpheta or Peumus genus for the production of cosmetic preparations or as active agent for producing a composition for increasing the metabolic activity of glucose-6-phosphate dehydrogenase (G6PDH).

EP 0045837 A1 discloses iridoid derivatives and the process for preparing them from catapol as an easily obtainable natural substance as well as their use as intermediates for the manufacture of prostanoids.

Bianco A. et al., in Tetrahedron Letters, 1978, vol. 48, 4829-4832 disclose iridoid aglycones, their reactivity and instability in acid medium.

Khobrakova V.B. et al. in Pharmaceutical Chemistry Journal, 2017, vol. 51, no. 5, 384-387, disclose plant dry extracts from Gentiana *algida* Pall. Having immunomodulating activity.

Hoki Kunihito et al. in Journal of Physical Chemistry B, 2004, vol. 108, no. 15, 4916-4921, disclose a study on a chiral molecular motor.

Kunihito Hoki et al. in Journal of Chemical Physics, 2003, vol. 119, no. 23, 12393-12398, report a study on a molecular motor in which a formyl group is the rotating part of the motor.

Schloegl K. et al. in Monatshefte fuer chemie, 1964, vol. 95, no. 2, 558-575, disclose the preparation of asymmetric amines and aldehydes of the ferrocene series.

Benkeser Robert A. et al. in Journal of the American Chemical Society, 1964, vol. 86, no. 5, 890-895, report a study on the metalation of alkylferrocene.

US 3703543 A discloses hydroxamic acids of alicyclic amino acids and esters thereof having a suppressive action of the immune response in warm-blooded animals.

Orhan Ilkay Erdogan et al. in Industrial Crops and Products, 2016, vol. 81, 117-121, report a study on the ability of selected plant essential oils to modulate the human cellular immune responses using various inflammatory parameters

such as reactive oxygen species (ROS) generation from whole blood phagocytes and isolated polymorphonuclear neutrophils (PMNs).

Kobayashi Yuko et al. in Bioscience, Biotechnology and Biochemistry, 2016, vol. 80, no. 6, 1172-1178, report a study on the anti-oxidant and anti-inflammatory effects of geranium essential oil as well as its role in allergic reactions.

Lee Je-Hyuk et al. in Food Science and Biotechnology, 2009, vol. 18, no. 6, 1371-1378, report a study on anti-inflammatory activities of *Z.piperitum* essential oil.

Lee Je-Hyuk et al. in Journal of the Science of Food and Agriculture, 2009, vol. 89, no. 10, 1762-1769, disclose a study on *Z.schinifolium* essential oil and its use as an alternative medicine for the relief and retardation of immunological inflammatory responses.

DE 19644422 A1 discloses the use of terpene compounds for immunosuppressive, anti-leukemia and anti-retroviral treatments.

Yu Xia et al. in Journal of Chinese Pharmaceutical Sciences, 2009, vol. 18, no. 3, 232-235, disclose a study on the extracts of a Chinese mangrove plant Cerbera manghas L. containing a new linear monoterpene.

EP 1371372 A1 discloses the use of mixtures of tocopherols and extracts of Harpagophytum procumbens (or active agents of the extract selected from iridoid glucosides, harpagosides, harpagides and procumbides) in the production of medicaments for combatting rheumatoid arthritis.

Elmasri Wael A. et al. in Rapid Communications in Mass Spectrometry, 2016, vol. 30, no. 18, 2033-2042, disclose a method for separating and characterizing iridoid glucosinolates by permethylation combined with LC/MS analysis.

Baden Christian Ulrich et al. in Chemoecology, 2012, vol. 22, no. 2, 113-118, report an investigational study on several species of the weevil family Mecininae that all feed on plants of the Plantaginaceae containing iridoid glycoside.

Akkol Esra Kupeli et al. in Pharmaceutical Biology, 2009, vol. 47, no. 3, 188-194, disclose a study on the effect of administration in mice of plant extracts from *Linaria aucheri.*

Viccini et al. in Journal of Molecular Structure, 2008, vol. 875, no. 1-3, 27-31, report the structural and spectroscopic characterization of ipolamiide and fulvoipolamiide isolated from the leaves of *Stachytarpheta glabra.*

Leitao Gilda Guimaraes et al. in Journal of Liquid Chromatography & Related Technologies, 2005, vol. 28, no. 12-13, 2053-2060, report a study on the glycosylated phenylpropanoids and iridoids isolated from the ethyl acetate extract from the roots of *Stachytarpheta cayennensis.*

Schapoval Elfrides E.S. in Journal of Ethnopharmacology, 1998, vol. 60, no. 1, 53-59, report a study on the anti-inflammatory properties and antinociceptive activity of the alcoholic extracts of the dried leaves of *Stachytarpheta cayennensis.*

## Summary of the invention

The present invention is as defined in the appended claims.

The present invention provides compounds derived from ipolamiide, which have immunosuppressive activity. Therefore, they can be used for the treatment of immunological disorders. Additionally, the present invention describes vegetal extracts enriched with said compounds, derived from ipolamiide, obtained through the present process of production, also having immunosuppressive activity.

It is, therefore, an object of the present invention ipolamiide derivatives that are compounds of the general formula C:

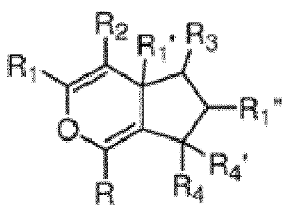

**C**

wherein R corresponds to H, OH, OGlyc (Glycoside); $R_1$, $R_{1'}$, $R_{1''}$ correspond to H, OH; $R_2$ corresponds to H, COOH, $COOCH_3$, $CH_3$, CHO; $R_3$ corresponds to H, OH, $CH_3$; $R_4$, $R_{4'}$ correspond to H, OH, $CH_2OH$, $CH_3$.

In a preferred embodiment, at least one preferred compound of the present invention may be selected from the group comprising the following structures:

It is also an object of the present invention a compound of formula C for use as immunosuppressant in the treatment of immunological disorders. In a preferred

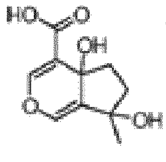

(IV)

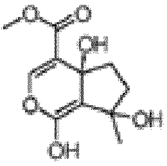

(V)

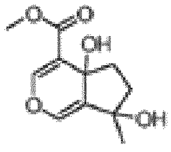

(VIII)

embodiment, the compound of formula C for use is intended for the treatment of vitiligo.

Furthermore, it is an object of the present invention a pharmaceutical composition for the treatment of immunological disorders, comprising at least one compound of formula C and a pharmaceutically acceptable vehicle.

In an optional embodiment, the composition of the present invention further comprises the ipolamiide compound.

As previously mentioned, we verified that isolated ipolamiide does not demonstrate immunosuppressive activity. On the other hand, certain groups of compounds derived from ipolamiide have such activity. At the same time, we have also specified the advantages of obtaining herbal medicines for the treatment of diseases, since these compound production systems allow a series of productive interactions between the components of the plant and the active compounds, often even synergistically. Thus, to additionally obtain an herbal medicine comprising such active compounds, we have developed a production process, which allows obtaining an extract enriched with compounds of interest. As described below, the extract production process of the present invention comprises steps that lead to extracts enriched with the ipolamiide derivatives with immunosuppressive activity. We verified the relevance of preselecting input vegetal biomasses containing between 2.5% and 3.5% of ipolamiide, resulting in an extract enriched with ipolamiide and compounds derived from ipolamiide from about 1% to about 20%, preferably from about 8.5% to about 11.5% of content of ipolamiide and derivatives.

As previously presented, the vegetal biomass containing this compound will be used as starting material for the production process of the extract. Only with the production process of the present invention it is possible to obtain an extract enriched with specific compounds derived from ipolamiide. This enriched extract, further, presents immunosuppressive activity.

It is, therefore, an additional object of the present invention a process for production of extract enriched with compounds derived from ipolamiide of formula C, comprising essentially the steps of:

a) selecting input vegetal biomass with a content of ipolamiide between 2.5% and 3.5% obtained from plants of the genus *Stachytarpheta;*
b) submitting the selected biomass from a) to oven drying at temperature between 40 to 80 °C, until obtaining the humidity stabilization between 10 to 12%;
c) milling the vegetal biomass;
d) performing the extraction of the vegetal biomass through the steps of:

   i. heating of the vegetal biomass at a temperature between 70 to 100 °C, with constant stirring;
   ii. maceration of the vegetal biomass at room temperature;
   iii. heating of the vegetal biomass with temperature between 70 to 100 ºC.

In a preferred embodiment, the process for production of the present invention further comprises the steps of:

iv. filtering and concentration of the extract;
v. drying in Spray Dryer, during 1 to 60 seconds, with inlet temperature between 155 and 165 °C and outlet temperature between 85 to 95 °C, coupled to a dehumidifier.

In a preferred embodiment, the process for extraction of the present invention is an aqueous or hydroalcoholic process, even more preferably aqueous process.

Therefore, the process for production of the present invention allows to obtain a standardized extract enriched with compounds derived from ipolamiide, preferably with a yield of about 8% to about 10%.

It is, therefore, an additional object of the present invention the extract enriched with compounds derived from ipolamiide obtained by the above-mentioned procedure.

The vegetal biomass of the present invention comprises all parts of plants of the genus *Stachytarpheta.* Preferably, the vegetal biomass comprises the aerial parts of the plants, more preferably, the leaves.

In a preferred embodiment, the input vegetal biomass comprises at least one vegetal biomass with uniform content of ipolamiide between 2.5% and 3.5%. In an optional embodiment, the input vegetal biomass comprises more than one vegetal biomass, wherein the different vegetal biomasses have different contents of ipolamiide independently,

but together achieve an uniform content of ipolamiide (between 2.5% and 3.5%).

In another preferred embodiment, the actual content of ipolamiide in the input vegetal biomass can be used as a parameter for predicting the theoretical content of ipolamiide and derivatives in the extract obtained. This prediction can be accomplished by a method comprising the step of applying Equation I to some parameters obtained experimentally to find the ideal proportions of ipolamiide in the input vegetal biomass, which preferably projects the content of ipolamiide and derivatives in the extract from 8.5% to 11.5% of. The Equation I is defined below:

% *Theoretical content of ipolamiide and derivatives in the extract* = % *Actual content of ipolamiide in the vegetal biomass x DER / (< actual content of ipolamiide and derivatives in the extract/actual content of ipolamiide in the input vegetal biomass >) ± standard deviation (Equation I).*

In this way, it is possible to predict the theoretical content of ipolamiide and derivatives in the extract from the actual content of ipolamiide in the input vegetal biomass. Preferably, the ratio between the actual content of ipolamiide and derivatives in the extract/content of ipolamiide in the input vegetal biomass is between about 3.0 and about 3.5.

In an embodiment even more preferred, the plants of the present invention comprise *Stachytarpheta cayennensis.*

It is, therefore, an additional object of the present invention the extract enriched with compounds of formula C derived from ipolamiide, obtained from plants of the genus *Stachytarpheta* for use as a medicament with immunosuppressive activity.

It is, therefore, an additional object of the present invention at least one active fraction of extract enriched with compounds derived from ipolamiide. Preferably, at least one fraction comprises at least one compound derived from ipolamiide of formula (I), (II) and/or (III).

In an optional embodiment, the active fraction of enriched extract further comprises ipolamiide.

It is, therefore, an additional object of the present invention the use of at least one standardized fraction enriched with compounds derived from ipolamiide, obtained from plants of the genus *Stachytarpheta* for the manufacture of a medicament with immunosuppressive activity.

These and other objects of the invention will be readily appreciated by those skilled in the art and by the companies having interests in the segment, and will be described in sufficient detail for its reproduction in the following description.

## Detailed description of the figures

Figure 1 - Summary flowchart describing the production process of the active extract enriched with ipolamiide derivatives obtained from *Stachytarpheta cayennensis.*

Figure 2 - Effect of the aqueous extract of *Stachytarpheta cayennensis* (3, 10 and 30 μM, concentration expressed in ipolamiide) and isolated ipolamiide (3, 10 and 30 μM) on the proliferation of CD8+ T cells activated by αCD3/CD28 (A) and IFNγ production (B). The effect of the pool of compounds (IV to VIII) and the five isolated compounds (IV to VIII) generated after the acid hydrolysis of ipolamiide (30 μM) was also evaluated in the same experiments, proliferation of CD8+ T cells activated by αCD3/CD28 (C) and IFNγ production (D). Tacrolimus (0.5 μM) was used as the positive control for all experiments. The data are the mean ± SD of three replicates.

Figure 3 - Effect of the acid hydrolysis of ipolamiide on the formation of its derivatives. Chromatogram of intact ipolamiide (blue); Ipolamiide hydrolyzed at 0.1 N HCl at 40 °C for 1h (green); Ipolamiide hydrolyzed at 0.1 N HCl at 40 °C for 2h (red); Ipolamiide hydrolyzed at 0.1 N HCl at 40 °C for 5h (magenta). IPO = Ipolamide.

Figure 4 - Chromatogram of the *Stachytarpheta cayennensis* extract obtained from the production process claimed herein. The figure illustrates the ipolamiide marker and its specific derivatives at retention times: 5.5; 9.7; 12.0; 14.3; 17.3 min. IPO = Ipolamiide.

## Detailed description of the invention

### Active compounds

The present description presents compound groups, comprising the following general formulas:

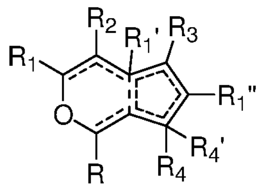
(I) ,
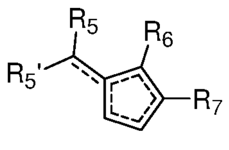
(II) ,
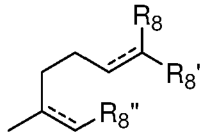
(III)

wherein R corresponds to H, OH, OGlyc (Glycoside); $R_1$, $R_{1'}$, $R_{1''}$ correspond to H, OH; $R_2$ corresponds to H, COOH, $COOCH_3$, $CH_3$, CHO; $R_3$ corresponds to H, OH, $CH_3$; $R_4$, $R_{4'}$ correspond to H, OH, $CH_2OH$, $CH_3$; $R_5$, $R_{5'}$ correspond to H, $CH_3$, $COOCH_3$, CHO, $CH_2OH$; $R_6$ corresponds to CHO, COOH, $COOCH_3$; $R_7$ corresponds to H, $CH_3$; Rs, $R_{8'}$, $R_{8''}$ correspond to CHO, $CH_3$, $CH_2OH$, COOH and the dashed bonds represent single (C-C) or double (C=C) bonds between carbons (up to two double bonds per structure).

Example 1: The compounds of general formula (I) comprise the following structures.

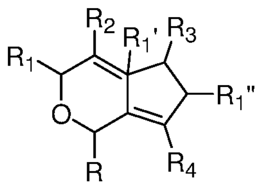
**A**
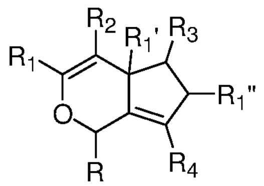
**B**
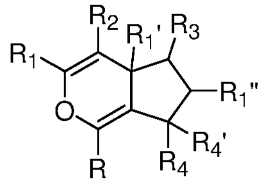
**C**

Example 2: The compounds of general formula (II) comprise the following structures.

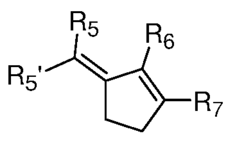
**D** ,
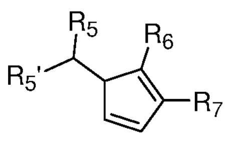
**E** ,
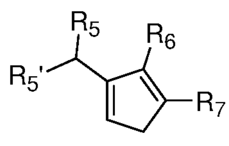
**F**
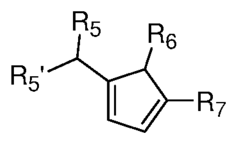
**G**

Example 3: The compounds of general formula (III) comprise the following structures.

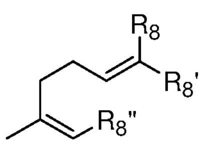
**H**
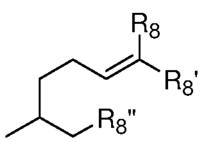
**I**
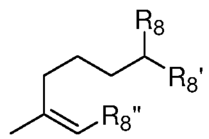
**J** .

Those above mentioned structures exemplify chemical structures backbones included in formulas (I), (II) or (III).

Immunological disorders

The term "immune disorders" of the present invention comprises any dysfunction of the immune system. Commonly the disorders can be characterized by the components of the immune system that are affected or by the level of activity of the immune system. In the present invention, preferably, the immune disorders refer to diseases that have some evidence of autoimmunity. We can consider in the present invention vitiligo as being even more preferably chosen among the possible immune disorders.

Method of Treatment of Immune Disorders

The present description provides a method of treatment of immune disorders, comprising administering to a patient a compound of general formula (I), (II) and/or (III), in sufficient amount to provide immunosuppressive effect. It should be noted that, for the purpose of the present patent application, the treatment of immune disorders can be achieved using ipolamiide derivatives and/or fractions and/or extracts containing ipolamiide derivatives, any of these having immunosuppressive activity. In a preferred aspect of the description, the method of treatment is intended for the treatment of vitiligo.

Pharmaceutical composition comprising isolated compounds derived from ipolamiide.

In one aspect of the description, the pharmaceutical composition comprises isolated ipolamiide derivatives, used alone or in combination, for the treatment of immune disorders, wherein the compounds comprise at least one compound selected from the group comprising:

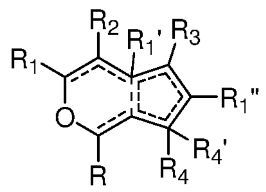

a) **(I)** ,

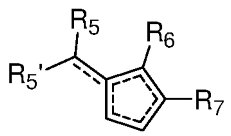

b) **(II)** ;

and/or

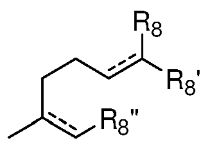

c) **(III)** ,

wherein R corresponds to H, OH, OGlyc (Glycoside); $R_1$, $R_{1'}$, $R_{1''}$ correspond to H, OH; $R_2$ corresponds to H, COOH, $COOCH_3$, $CH_3$, CHO; $R_3$ corresponds to H, OH, $CH_3$; $R_4$, $R_{4'}$ correspond to H, OH, $CH_2OH$, $CH_3$; $R_5$, $R_{5'}$ correspond to H, $CH_3$, $COOCH_3$, CHO, $CH_2OH$; $R_6$ corresponds to CHO, COOH, $COOCH_3$; $R_7$ corresponds to H, $CH_3$; Rs, $R_{8'}$, $R_{8''}$ correspond to CHO, $CH_3$, $CH_2OH$, COOH and the dashed bonds represent single (C-C) or double (C=C) bonds between carbons (up to two double bonds per structure); and

d) pharmaceutically acceptable vehicle.

In an optional embodiment, the present pharmaceutical composition further comprises the ipolamiide compound.

In a preferred aspect of the description, the pharmaceutical composition comprises the compounds:

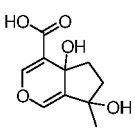 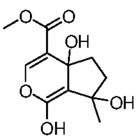 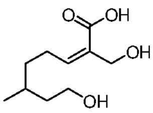 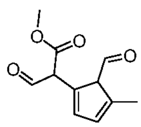 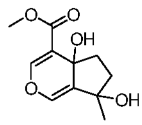 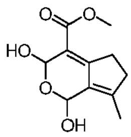 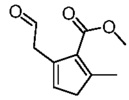 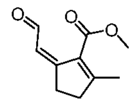

(IV) (V) (VI) (VII) (VIII) (IX) (X) (XI)

In this aspect of the description, the compound of general formula (I) comprises the compounds of formula (IV), (V),(VIII) and (IX), the compound of general formula (II) comprises the compound of formula (VII), (X) and (XI) and the compound of general formula (III) comprises the compound of formula (VI).

The pharmaceutical composition of the present invention may additionally comprise the following compounds:

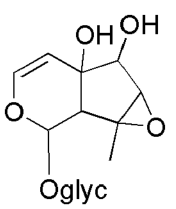 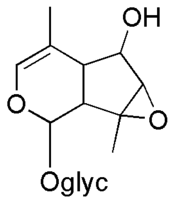 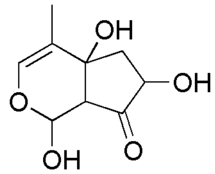 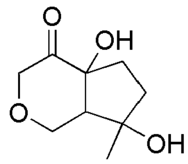 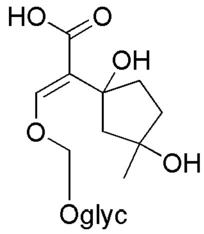

(XII) (XIII) (XIV) (XV) (XVI) ;

and pharmaceutically acceptable vehicle.

Pharmaceutically acceptable vehicle.

To carry out their activity, the compounds of general formula (I), (II) and/or (III) should be administered to an animal organism, a mammal, particularly a human, preferably in the form of a pharmaceutical composition, i.e., associated with pharmaceutically acceptable vehicles which are suitable for each route of administration.

The pharmaceutical compositions of the present invention contain as active ingredient one or more compounds proposed herein, associated with one or more pharmaceutically acceptable vehicles. The active ingredient is commonly mixed, diluted or encapsulated with at least one vehicle.

When the vehicle is a diluent, it may be in the solid, semi-solid or liquid form, acting as a carrier, excipient or medium for the active ingredient. Therefore, the composition can be in the form of tablets, pills, powders, sachets, suspensions, emulsions, solutions, aerosols (in solid or liquid medium), creams, hard or soft capsules, suppositories, injectable solutions.

In the present invention, it is preferably considered as pharmaceutically suitable vehicle any substance different from the compound of general formula (I), (II) or (III), which has been intentionally added thereto to produce a pharmaceutical dosage form appropriate to a route of administration. Non-limiting examples of pharmaceutical excipients suitable for the preparation of pharmaceutical compositions are described in Handbook of Pharmaceutical Manufacturing Formulations - Vol. 1 to 6 - 2004 - Sarfaraz K. Niazi - CRC Press e Remington's Pharmaceutical Sciences, Mack Publishing.

Non-limiting examples of routes of administration of the composition comprising the compound of general formula (I), (II) or (III) are the topical, oral, parenteral, nasal, rectal, transmucosal, transdermal routes.

The therapeutic dose to be employed of the compounds of the present invention should be planned and calculated according to the route of administration chosen, the age, the weight and condition of the patient and the severity of the treated disorder. In general, the compounds of the present invention are administered in therapeutically effective doses. Effective doses can be extrapolated from doseresponse curves, derived from *in vitro* or animal models. Typically, the clinician will administer the compound until an appropriate dose to achieve the desired effect.

Process of Production of Active Compounds

The present description illustrates in detail the process of production of active compounds of general formula (I), (II) and/or (III). Essentially, the process of production of isolated compounds comprises the step of subjecting at least one ipolamiide compound to at least one heating step at high temperatures, in suitable solvent, for a sufficient time to obtain the compounds derived from ipolamiide of the general formula (I), (II) and/or (III).

In an optional embodiment, the composition of the present invention further comprises the ipolamiide compound.

In a preferred embodiment, the solvent of the present invention comprises water.

Additionally, the present description provides the process of production of the compounds of general formula (I), (II) and/or (III), comprising the step of subjecting at least one ipolamiide compound to a heating step at high temperatures, in suitable solvent, for a sufficient time to obtain the compounds derived from ipolamiide of the general formula (I), (II) and/or (III).

In a preferred embodiment, high concentrations of the compounds of formula (I), (II) and/or (III) of the present description are obtained from the total conversion (100%) of the content of ipolamiide used in the respective production process.

Even more preferably, the high concentrations of the present description comprise about 0.5% to about 45% of each compound (IV to VIII) in a mixture. In this case, the concentrations comprise about 1 to about 5% of the compound IV, preferably 4%; about 15 to about 25% of the compound V, preferably 19%; about 1% to about 6% of the compound VI, preferably 3%; about 35% to about 45% of the compound VII or its isomers (e.g. compound X or compound XI), preferably 37%; about 0.5% to about 4% of the compound VIII or its isomers (e.g. compound IX), preferably 2%.

In a preferred embodiment, the high temperatures of the present invention comprise temperatures above 35 °C, more preferably between 35 °C and 165 °C.

In another preferred embodiment, the solvent of the present invention comprises other suitable solvents.

In a preferred embodiment, the process of production of the active compounds derived from ipolamiide comprises the step of hydrolysis or solvolysis of ipolamiide.

Even more preferably, the hydrolysis of ipolamiide may be of the acid type. Among the acids suitable for acid hydrolysis of ipolamiide, hydrochloric acid, hydrochloric, sulfuric, nitric, phosphoric and acetic acid can be mentioned, without any specific restrictions to any of them. In a preferred embodiment, in the present invention hydrochloric acid is used.

In an optional embodiment, the hydrolysis of ipolamiide may be of the basic/alkaline type. Among the bases suitable for basic/alkaline hydrolysis of ipolamiide, the alkali-metal hydroxides can be mentioned, without specific restrictions to any of them. In a preferred embodiment, in the present invention sodium hydroxide is used.

In an even more preferred embodiment, the hydrolysis in acidic medium is followed by a hydrolysis in basic/alkaline medium. As an example, we can mention the production process comprising a step of submitting at least one ipolamiide compound to high temperatures and 0.1 N (eq/L) of hydrochloric acid, followed by incubation for different time intervals that can range from 0 to 120 minutes. The hydrolysis is then interrupted by a process of neutralization using, preferably, sodium hydroxide 0.1 N.

In an optional embodiment, the production process of the compounds of general formula (I), (II) and/or (III) comprises a step of hydrolysis of ipolamiide in basic medium, being carried out with aqueous sodium hydroxide solution 0.1 N, maintained at 40 °C for 2 hours.

Production process of active fractions/extracts enriched with ipolamiide derivatives

It was verified by the inventors that certain isolated active compounds of the present invention can be obtained by techniques of molecular *design* and synthesis. At the same time, we have also specified the advantages of obtaining herbal medicines for the treatment of diseases, since these compound production systems allow a series of productive interactions between the components of the plant and the active compounds, often even synergistically. Thus, to additionally obtain an herbal medicament comprising such active compounds, we have developed a production process, which allows obtaining an extract enriched with compounds of interest.

As described below, the extract production process of the present invention comprises steps that lead to extracts enriched with the ipolamiide derivatives with immunosuppressive activity. We verified the relevance of preselecting input vegetal biomasses containing between 2.5% and 3.5% of ipolamiide, resulting in an extract enriched with ipolamiide and compounds derived from ipolamiide from about 8.5% to about 11.5%. As previously presented, the vegetal biomass containing ipolamiide will be used as starting material for the production process of the extract. Only with the production process of the present invention it is possible to obtain an extract enriched with specific compounds derived from ipolamiide. This enriched extract, further, presents immunosuppressive activity.

Thus, the present invention provides a process to produce standardized extract enriched with ipolamiide derivatives from plants of the genus *Stachytarpheta.*

It is, therefore, an additional object of the present invention a process for production of extract enriched with compounds derived from ipolamiide, comprising essentially the steps of:

a) selecting input vegetal biomass with a content of ipolamiide between 2.5% and 3.5% obtained from plants of the genus *Stachytarpheta;*

b) submitting the selected biomass from a) to oven drying at temperature between 40 to 80 °C, until obtaining the humidity stabilization between 10 to 12%;

c) milling the vegetal biomass;
d) performing the extraction of the vegetal biomass through the steps of:

i. heating of the vegetal biomass at a temperature between 70 to 100 °C, with constant stirring;
ii. maceration of the vegetal biomass at room temperature;
iii. heating of the vegetal biomass with temperature between 70 to 100 ºC.

In a preferred embodiment, the process for production of the present invention further comprises the steps of:

iv. filtering and concentration of the extract;
v. drying in Spray Dryer, during 1 to 60 seconds, with inlet temperature between 155 and 165 °C and outlet temperature between 85 to 95 °C, coupled to a dehumidifier.

In a preferred embodiment, the process for extraction of the present invention is an aqueous or hydroalcoholic process, even more preferably an aqueous process.

Therefore, the process for production of the present invention allows obtaining a standardized extract enriched with compounds derived from ipolamiide, preferably with a yield of about 8% to about 10%.

It is, therefore, an additional object of the present invention the extract enriched with compounds derived from ipolamiide obtained by the above-mentioned procedure. The standardized extract enriched with compounds derived from ipolamiide of the present description comprises, preferably, the compounds of formula (I), (II) and/or (III).

The vegetal biomass of the present invention comprises all parts of plants of the genus *Stachytarpheta.* Preferably, the vegetal biomass comprises the aerial parts of the plants, more preferably, the leaves.

In a preferred embodiment, the input vegetal biomass comprises at least one vegetal biomass with uniform content of ipolamiide between 2.5% and 3.5%. In an optional embodiment, the input vegetal biomass comprises more than one vegetal biomass, wherein the different vegetal biomasses have different contents of ipolamiide independently, but together achieve an uniform content of ipolamiide (between 2.5% and 3.5%).

In another preferred embodiment, the actual content of ipolamiide in the input vegetal biomass can be used as a parameter for predicting the theoretical content of ipolamiide and derivatives in the extract obtained. This prediction can be accomplished by a method comprising the step of applying Equation I to some parameters obtained experimentally to find the ideal proportions of ipolamiide in the input vegetal biomass, which preferably projects the content of ipolamiide and derivatives in the extract from 8.5% to 11.5% of. The Equation I is defined below:

% *Theoretical content of ipolamiide and derivatives in the extract* = % *Actual content of ipolamiide in the vegetal biomass x DER / (< actual content of ipolamiide and derivatives in the extract/actual content of ipolamiide in the input vegetal biomass >) ± standard deviation (Equation I).*

In this way, it is possible to predict the theoretical content of ipolamiide and derivatives in the extract from the actual content of ipolamiide in the input vegetal biomass. Preferably, the ratio between the actual content of ipolamiide and derivatives in the extract/content of ipolamiide in the input vegetal biomass is between about 3.0 and about 3.5.

The present invention further claims standardized extracts enriched with compounds derived from ipolamiide from plants of the genus *Stachytarpheta* obtained from the production process described above.

In an embodiment even more preferred, the plants of the present invention comprise *Stachytarpheta cayennensis.*

The standardized extracts from plants of the genus *Stachytarpheta* are preferably obtained by the production process described above resulting in an extract enriched with ipolamiide and compounds derived from ipolamiide from about 1% to about 20%, preferably from about 8.5% to about 11.5% of content of ipolamiide and derivatives.

It is, therefore, an additional object of the present invention the use of standardized extracts of plants of the genus *Stachytarpheta* containing compounds derived from ipolamiide for the manufacture of a medicament with immunosuppressive activity. More specifically, standardized extracts of plants of the genus *Stachytarpheta* containing compounds derived from ipolamiide of general formula (I), (II) and/or (III) for the manufacture of a medicament with immunosuppressive activity.

It is, therefore, an additional object of the present invention at least one active fraction of extract enriched with compounds derived from ipolamiide. Preferably, at least one fraction comprises at least one compound derived from ipolamiide of formula (I), (II) and/or (III).

In an optional embodiment, the active fraction of enriched extract further comprises ipolamiide.

It is, therefore, an additional object of the present invention the use of at least one standardized fraction enriched with compounds derived from ipolamiide, obtained from plants of the genus *Stachytarpheta* for the manufacture of medicament with immunosuppressive activity.

Examples - Preferred Embodiment

Process of Production and Identification of Active Compounds

The isolated compounds of the present invention are obtained by subjecting the ipolamide compound to 0.1 N hydrochloric acid, at 40 °C and 100 °C, for 1h, 2h and 5h. Figure 3 illustrates the condition at 40 °C. From this experiment, we could observe several products from this hydrolysis and, based on chromatograms, identify several derivatives of ipolamiide, such as, for example, the structures described below, as illustrated on figure 3.

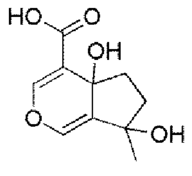
(IV)

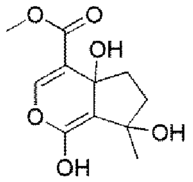
(V)

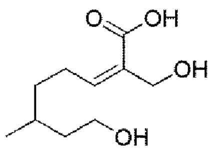
(VI)

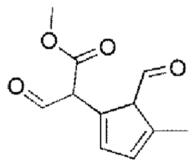
(VII)

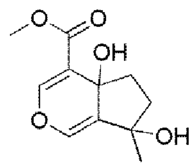
(VIII) .

Alternatively, the hydrolysis can be carried out by varying the hydrochloric acid concentration between 0.1 to 1 N and the experimental temperature may vary between 35 °C and 165 °C. In addition, the hydrolysis time can vary between 1 minute and 24 hours to facilitate the formation of higher concentrations of certain ipolamiide derivatives.

Process for obtaining the extract

According to the present invention, the process for obtaining aqueous extract of *Stachytarpheta cayennensis* rich in ipolamiide derivatives mainly comprised the steps of producing a standardized extract illustrated in the flowchart of Figure 1 to obtain material for the development of pre-clinical research in immunology.

At first, seeds preferably selected by genotyping of *Stachytarpheta cayennensis* were submitted to a process of seeding for two months in a controlled environment regarding temperature, humidity and light. The seeding was carried out in expanded polystyrene trays, filled with substrate, and kept in this protected environment with controlled irrigation. The seedlings began to appear in 10 to 15 days. The trays remained in these conditions until the seedlings reached size and ideal conditions for permanent transplantation.

The seedlings with an approximate size of 5 to 8 cm in height and with 2 to 3 pairs of definitive leaves were transplanted to the growing site, which preferably had an annual average temperature of 30ºC, annual average relative humidity of less than 55%, and in which the soil had preferably, but not limited to, the results of specific chemical and physical soil analyses, for example, acidity, calcium, nitrogen and use of organic fertilization in all areas.

After planting the seedlings, the first harvest was carried out after 6 months, and the other regrowth every 4 months, thus guaranteeing an optimized life cycle for the shrub aiming at maximizing ipolamiide content in the input vegetal biomass.

Following the planting, the vegetal biomass was stabilized through a greenhouse drying process, under defined conditions of temperature and humidity. The plants were dried in dryers with heat exchanger, forced air circulation, and temperature ranging from 50 to 70 °C, preferably 60 °C. The drying consisted of the passage of hot air through the plants, removing the humidity, until the vegetal biomass was stabilized with humidity between 10 to 12%. In a preferred embodiment, the drying time occurs from 8 to 20 hours.

As shown in Figure 1, the vegetal biomass was subjected to the milling process by means of a hammer mill with 1800RPM and a 19mm sieve, obtaining a productivity of 50 to 150 kg/hour at room temperature. After milling, the vegetal biomass was submitted to an aqueous extraction at temperature between 80 to 90 °C, with constant stirring for 15 min. The amount of extractive solution used should be 10× the amount of vegetal biomass used, guaranteeing exhaustive extraction of the substance of interest in the vegetal biomass.

After the previous step, the material was submitted to the maceration process for 10h at room temperature. Subsequently, the material was again heated at 80 to 90 °C for 15 min.

The material was filtered on a rotary filter with polyester mesh of 40um. After the filtration step, the material was concentrated on a "Bernauer" evaporator and/or falling film evaporator to about 30% of total solids.

The product of this step was submitted to drying in Spray Dryer, with inlet temperature between 155 to 165 °C and outlet temperature between 85 to 95 °C coupled to a dehumidifier, preferably of the Bry-Air type, during 20 to 40 seconds, aiming at obtaining the lowest residual humidity content possible (Table 1). This process substantially improves the quality of the enriched extract, since the residual humidity in the material initially compromises the stability of the components of interest, during the shelf life of the vegetal extract/derivative. This extractive process resulted in a ratio of 10 to 12:1 and yield varying between 8 to 10%.

Table 1 - Humidity content in the extract after drying only and after drying with humidifier

| Humidity content in the extract after drying with SD | Humidity content in the extract after drying with SD + dehumidifier |
|---|---|
| 4.61% | 1.77% |

The extractive process described above guaranteed the exhaustive extraction of ipolamiide to assure the immunosuppressive activity of the extract due to the presence of ipolamiide derivatives generated in the process. Therefore, it was necessary to establish process controls for the input vegetal biomass, so that the actual content of ipolamiide and derivatives in the extract was between 8.5 and 11.5%, as follows in Table 2. The control of ipolamiide in the vegetal biomass ensures the presence of ipolamiide derivatives with immunosuppressive activity in the extract, generated by the process described herein.

Table 2 - Content of ipolamiide in the vegetal biomass and in the extract.

| Actual content of ipolamiide in the input vegetal biomass | Theoretical content of ipolamiide and derivatives in the extract | Actual content of ipolamiide and derivatives in the extract[a] | Selection of input vegetal biomass |
|---|---|---|---|
| 1.57% | 5.23% (4.44 - 6.02) | 4.47% | Not selected |
| 2.20% | 7.33% (6.23 - 8.43) | 7.62% | Not selected |
| 3.00% | 10% (8.5 - 11.5) | 10.80% | Selected |
| 3.30% | 11% (9.35 - 12.65) | 11.20% | Selected |
| 3.07% | 10.2% (8.67 - 11.73) | 8.90% | Selected |
| 3.65% | 12.2% (10.37 - 14.03) | 12.40% | Not selected |
| [a]The agreement between the theoretical and actual contents of ipolamiide and its derivatives in the extract demonstrates the applicability of Equation I. | | | |

With this, it was possible to determine a method for predicting the theoretical content of ipolamiide and its derivatives in the enriched active extract. For this purpose, it was used an equation to project the content that would be obtained by the extraction process (theoretical) from the actual content of ipolamiide in the vegetal biomass (Equation I).

% *Theoretical content of ipolamiide and derivatives in the extract* = % *Actual content of ipolamiide in the vegetal biomass x DER / (< actual content of ipolamiide and derivatives in the extract/actual content of ipolamiide in the input vegetal biomass >) ± standard deviation (Equation I).*

In the above-described equation, DER can be understood as the amount of vegetal biomass required to obtain 1 kilo of native extract. The ratio between the actual content of ipolamiide and derivatives on the extract/content of ipolamiide in the input vegetal biomass is, preferably, between about 3.0 and about 3.5.

Thus, in a prospective way, using the above equation, it is possible to select only the vegetal biomass (whose content of ipolamiide is obtained by analytical methods, such as HPLC) that projects an adequate theoretical content of ipolamiide and derivatives in the extract between 8.5 and 11.5% and discard those that will not project this range. It has been verified in the present invention that the relationship between the actual content of ipolamiide and derivatives in the extract and the content of ipolamiide in the input vegetal biomass will, preferably, be between 3.0 and 3.5.

The HPLC analysis comprised the steps of preparing sample solutions and standards, and elution thereof, as follows:

1. Solutions preparation

1.1 - Formic acid solution 0.1% (mobile phase A): In a 1000 mL volumetric flask containing approximately 800 ml of ultra-pure water, it was added 1 ml of formic acid. The flask volume was filled with ultra-pure water and well homogenized.

1.2 - Formic acid diluent solution: methanol (1:1): In a beaker, it was mixed 50 mL of the 0.1% formic acid solution with 50 mL of methanol.

2. Sample preparation:

Raw-material (Herbal): 1.0 g of the ground herbal was weighed and transferred to an amber 250 ml Erlenmeyer flask or covered with aluminum foil. 50 mL of distilled water was added and extracted under reflux at 80 °C for 2 hours. The solution was paper-filtered into a 50 mL volumetric flask and filled up with distilled water. It was filtered through 0.22 OR 0.45 μm membrane to an HPLC vial.

3. Standards preparation:

Ipolamiide standard 100ppm: 1.0 mg of ipolamiide standard was weighed and transferred to an amber 10mL volumetric flask. 5 ml of the diluent was added, which was left in an ultrasonic bath for 10 minutes or until complete dissolution. It was filled with the diluent and then there was the homogenization. Humidity analysis by Karl Fischer was performed for the ipolamiide standard.

4. Analysis by HPLC:

4.1-PARAMETERS/EQUIPMENT

- Column: Zorbax SB-C18 (250 mm x 4 mm; 5um)
- Mobile phase:
(A) 0.1% formic Acid;
(B) Methanol.

Table 3 - Ipolamiide Elution Gradient

| Time (min) | (%) A | (%) B |
|---|---|---|
| 0 | 80 | 20 |
| 27 | 58 | 42 |
| 32 | 58 | 42 |
| 32.1 | 0 | 100 |
| 36 | 0 | 100 |
| 36.1 | 80 | 20 |
| 45 | 80 | 20 |
| - Flow rate: 1.0 mL/min<br>- Detection: 254 nm.<br>- Analysis time: 45 minutes<br>- Injection volume: 30 μL | | |

4.2 - CALCULATION OF IPOLAMIIDE CONTENT

Content of ipolamiide (%) = A sample x M standard x P standard x D sample x (100 - U standard);

A standard x M sample x D standard x 10000/ 100;

wherein:

A sample: Peak area of ipolamiide in the sample
M standard: Mass of the standard ipolamiide in mg
P standard: Purity of the standard in decimal
D sample: Dilution of the sample in mL
A standard: Peak area of ipolamiide in the standard
M sample: Mass of the sample used in g

D standard: Dilution of the standard in L
U standard: Humidity of the standard quantified by Karl Fischer
10000: Conversion of units

The result of the content in the extract was given in dry base, that is, the humidity was discounted. Therefore the calculation for the mass of the extract was:

$$M\ sample = Mass\ x\ (100 - U\ sample)/100;$$

wherein:

Mass = Mass of dry extract (in grams)
U sample = Humidity of the dry extract in percentage, according to iT2-052.

5. Analysis by HPLC for ipolamiide derivatives

5.1 - PARAMETERS/EQUIPMENTS

- Column: Eclipse XDB Agilent - C18 (150 X 4.6 MM) 5 MICRONS
- Mobile phase:
(A) 0.1% formic Acid buffer;
(B) Acetonitrile

Table 4 - Ipolamiide Derivatives Elution Gradient

| Time (min) | (%) A | (%) B |
|---|---|---|
| 0 | 95 | 5 |
| 5.00 | 90 | 10 |
| 8.00 | 80 | 20 |
| 10.00 | 90 | 10 |
| 12.00 | 95 | 5 |
| - Flow: 1.2 mL/min<br>- Detection: DAD (205-280nm).<br>- Analysis time: 15 minutes<br>- Injection volume: 20 μL | | |

5.2 CALCULATION OF CONTENT FOR IPOLAMIIDE DERIVATIVES

For analysis of a given ipolamiide derivative (Y), we submitted it to a solution with defined concentration. This concentration is directly correlated with the area observed in the chromatogram. This area, when compared with the total area of the chromatogram, multiplied by the concentration previously defined, give us the percentage value of the said derivative (Y) in the sample, as can be seen below:

$$\text{Sample concentration X } \frac{\text{Derivative Y area}}{\text{Total area}} \text{ X } 100 = \%\text{ of derivative Y content}$$

(Σ content of derivatives = % total content of ipolamiide derivatives in the sample.)

<u>Biological / immunosuppressive activity</u>

The evaluation of the *in vitro* biological activity of the aqueous extract of *Stachytarpheta cayennensis* as well as of ipolamiide and its derivatives was conducted as described below.

The extract of *Stachytarpheta cayennensis,* ipolamiide and derivatives were studied in an experimental *in vitro*

immunological model involving CD8+ T cells and IFNγ. The objective of this study was to evaluate whether the extract and other substances, isolated or mixed, act by blocking the activation of CD8+ T cells and the consequent secretion of IFNγ.

Peripheral blood mononuclear cells (PBMCs) from healthy volunteers were isolated from leukocyte layers by Ficoll-Hystopaque centrifugation. Thereafter, human CD8+ T cells were isolated using the CD8+ T cell isolation kit (Miltenyi Biotec, #130-096-495). These cells ($3\times10^5$ cells/well) were incubated in RPMI + 10% of FBS medium, activated with αCD3/CD28 (1 μg/mL) and treated with different concentrations of aqueous extract of *Stachytarpheta cayennensis* standardized in 10% of ipolamiide, isolated ipolamiide, and ipolamiide derivatives obtained by acid hydrolysis to study their effect on the prevention of the CD8+ T cell activation and secretion of IFNγ. For evaluation of cell proliferation, bromodeoxyuridine (BrdU), a thymidine analogue commonly used for proliferation assays, was used as a marker for the proliferation. Specifically, it was evaluated the incorporation of BrdU through the Biotrak ELISA System (GE Healthcare, RPN250) per the manufacturer's instructions. The methodology for quantification of interferon gamma comprised the use of the Human IFNγ ELISA Ready-SET-Go kit (eBiosciences 88-7316-88) and followed the manufacturer's instructions. As illustrated in the graph set of Figure 2, the aqueous extract of *Stachytarpheta cayennensis* enriched with ipolamiide derivatives blocked the activation of CD8+ T cells induced by αCD3/CD28. Isolated ipolamiide had no effect on the proliferation of CD8+ T cells induced by αCD3/CD28, or the secretion of IFNγ. However, the compounds derived from ipolamiide in a mixture obtained for its acid hydrolysis significantly reduced the CD8+ T cell proliferation and the secretion of IFNγ. The ipolamiide derivatives isolated from this mixture also demonstrated a statistically significant reduction of CD8+ T cell proliferation and of secretion of IFNγ. Tacrolimus, a well-known immunosuppressive agent, was used as a reference compound and as a positive control for the experiment. The effect obtained by tacrolimus was similar to that obtained with hydrolyzed ipolamiide in both cell proliferation and IFNγ production.

Thus, the results showed that the immunosuppressive activity derives from the ipolamiide derivatives and not from the intact molecule. It is important to reinforce that the compounds obtained by the experimental condition of acid hydrolysis of isolated ipolamiide are present in the aqueous extract of *Stachytarpheta cayennensis,* as shown in Figure 4. However, the presence of such compounds is due to the extraction process used in the present invention.

## Claims

1. Compounds of general formula C, which are ipolamiide derivatives

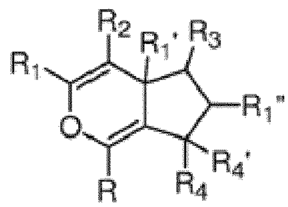

**C**

wherein R corresponds to H, OH, OGlyc (Glycoside); $R_1$, $R_{1'}$, $R_{1''}$ correspond to H, OH; $R_2$ corresponds to H, COOH, $COOCH_3$, $CH_3$, CHO; $R_3$ corresponds to H, OH, $CH_3$; $R_4$, $R_{4'}$ correspond to H, OH, $CH_2OH$, $CH_3$.

2. Compounds of formula C according to claim 1, selected from:

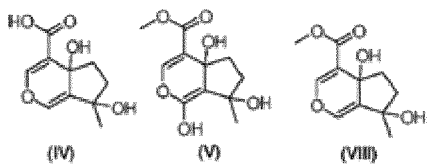

(IV) (V) (VIII)

3. Compounds of formula C as defined in claims 1 or 2, for use as a medicament.

4. Compounds of formula C as defined in claims 1 or 2, for use as immunosuppressants in the treatment of immune disorders.

**5.** Compounds of formula C as defined in claims 1 or 2, for use in the treatment of vitiligo.

**6.** A pharmaceutical composition comprising ipolamiide derivatives of formula C as defined in claims 1 or 2, and a pharmaceutically acceptable vehicle, optionally further comprising ipolamiide.

**7.** Process for production of extract enriched with ipolamiide derivatives of formula C as defined in claims 1 or 2, comprising the steps of:

a) selecting input vegetal biomass with a content of ipolamiide between 2.5% and 3.5% obtained from plants of the genus *Stachytarpheta;*
b) submitting the selected biomass from a) to oven drying at temperature between 40 to 80°C, until obtaining the humidity stabilization between 10 to 12%;
c) milling the vegetal biomass;
d) performing the extraction of the vegetal biomass through the steps of:

i. heating of the vegetal biomass at a temperature between 70 to 100°C, with constant stirring;
ii. maceration of the vegetal biomass at room temperature;
iii. heating of the vegetal biomass with temperature between 70 to 100°C.

**8.** Process for production of extract according to claim 7, further comprising the steps of:

iv. filtering and concentration of the extract;
v. drying in Spray Dryer, during 1 to 60 seconds, with inlet temperature between 155 and 165°C and outlet temperature between 85 to 95°C, coupled to a dehumidifier.

**9.** Process for production of extract, according to claim 7, in which the vegetal biomass heating of d) item iii) occur for 5 to 30 minutes.

**10.** Process for production of extract, according to claim 7, in which the vegetal biomass heating of d) item i) occur for 5 to 15 hours.

**11.** Process for production of extract, according to claim 7, in which the input vegetal biomass comprises the aerial parts of the plants of the genus *Stachytarpheta.*

**12.** Process for production of extract, according to claim 7, in which the plant is *Stachytarpheta cayennensis.*

**13.** Process for production of the extract, according to claim 7, in which the process comprises an aqueous extraction.

**14.** Process for production of extract, according to any one of claims 7 to 13, comprising the step of predicting the theoretical content of ipolamiide and its derivatives of formula C in the extract from the actual content of ipolamiide in the input vegetal biomass using Equation I:

$$\begin{matrix}\text{\% Theoretical content}\\ \text{of ipolamiide and de-}\\ \text{rivatives in the extract}\end{matrix} = \frac{\text{\% Actual content of ipolamiide in the vegetal biomass x DER}}{\frac{\text{(actual content of ipolamiide and derivatives in the extract)}}{\text{(actual content of ipolamiide in the input vegetal biomass)}}} \pm \text{standard deviation.}$$

Equation I

**15.** Extracts from plants of the genus *Stachytarpheta* enriched with ipolamiide derivatives of formula C as defined in claims 1 or 2.

**16.** Extracts from plants of the genus Stachytarpheta enriched with ipolamiide derivatives of formula C, obtained by the process as defined in any of the claims 7 to 14.

**17.** Extracts from plants of the genus *Stachytarpheta* according to claim 15 or claim 16, containing from 1% to 20% of ipolamiide or ipolamiide derivatives of formula C.

**18.** Extracts from plants of the genus *Stachytarpheta* as defined in any one of claims 15 to 17, for use as immunosuppressants in the treatment of immunological disorders.

## Patentansprüche

**1.** Verbindungen der allgemeinen Formel C, die Ipolamiid-Derivate sind

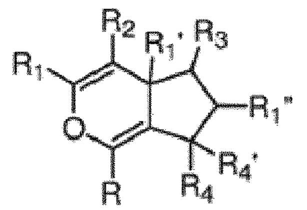

**C**

worin R entspricht H, OH, OGlyc (Glycosid); $R_1$, $R_{1'}$, $R_{1''}$ entspricht H, OH; $R_2$ entspricht H, COOH, $COOCH_3$, $CH_3$, CHO; $R_3$ entspricht H, OH, $CH_3$; $R_4$ entspricht H, OH, $CH_2OH$, $CH_3$.

**2.** Verbindungen der Formel C nach Anspruch 1, ausgewählt aus:

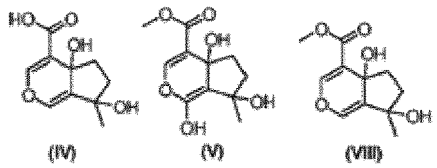

(IV) (V) (VIII)

**3.** Verbindungen der Formel C, wie sie in den Ansprüchen 1 oder 2 definiert ist, zur Verwendung als Arzneimittel.

**4.** Verbindungen der Formel C, wie sie in den Ansprüchen 1 oder 2 definiert ist, zur Verwendung als Immunsuppressiva bei der Behandlung von Immunerkrankungen.

**5.** Verbindungen der Formel C, wie sie in den Ansprüchen 1 oder 2 definiert ist, zur Verwendung bei der Behandlung von Vitiligo.

**6.** Pharmazeutische Zusammensetzung, die Ipolamiid-Derivate der Formel C, wie sie in den Ansprüchen 1 oder 2 definiert ist, und ein pharmazeutisch verträgliches Vehikel enthält, das gegebenenfalls weiterhin Ipolamiid enthält.

**7.** Verfahren zur Herstellung eines Extrakts, das mit Ipolamiid-Derivaten der Formel C angereichert ist, wie sie in den Ansprüchen 1 oder 2 definiert ist, das die Schritte enthält:

a) Auswahl von pflanzlicher Eingabe-Biomasse mit einem Gehalt an Ipolamiiden zwischen 2,5 % und 3,5 %, die aus Pflanzen der Gattung *Stachytarpheta* erhalten werden;
b) Unterziehen der ausgewählten Biomasse aus a) einer Ofentrocknung bei einer Temperatur zwischen 40 und 80 °C, bis eine Feuchtigkeitsstabilisierung zwischen 10 und 12 % erreicht ist;
c) Mahlen der pflanzlichen Biomasse;
d) Durchführen der Extraktion der pflanzlichen Biomasse durch die Schritte:

i. Erhitzen der pflanzlichen Biomasse auf eine Temperatur zwischen 70 und 100 ºC unter ständigem Rühren;
ii. Mazeration der pflanzlichen Biomasse bei Raumtemperatur;
iii. Erhitzen der pflanzlichen Biomasse mit einer Temperatur zwischen 70 und 100 ºC.

**8.** Verfahren zur Herstellung eines Extrakts nach Anspruch 7, das ferner die Schritte enthält:

iv. Filtrieren und Konzentrieren des Extrakts;

v. Trocknen im Sprühtrockner, während 1 bis 60 Sekunden, mit einer Einlasstemperatur zwischen 155 und 165 °C und einer Auslasstemperatur zwischen 85 und 95 °C, gekoppelt an einen Entfeuchter.

**9.** Verfahren zur Herstellung des Extraktes nach Anspruch 7, bei dem das Erhitzen der pflanzlichen Biomasse von d) Punkt iii) für 5 bis 30 Minuten erfolgt.

**10.** Verfahren zur Herstellung des Extraktes nach Anspruch 7, bei dem das Erhitzen der pflanzlichen Biomasse von d) Punkt i) für 5 bis 15 Stunden erfolgt.

**11.** Verfahren zur Herstellung des Extraktes nach Anspruch 7, bei dem die zugeführte pflanzliche Biomasse die oberirdischen Teile der Pflanzen der Gattung *Stachytarpheta* enthält.

**12.** Verfahren zur Herstellung des Extraktes nach Anspruch 7, wobei die Pflanze *Stachytarpheta cayennensis* ist.

**13.** Verfahren zur Herstellung des Extraktes nach Anspruch 7, wobei das Verfahren eine wässrige Extraktion umfasst.

**14.** Verfahren zur Herstellung des Extraktes nach einem der Ansprüche 7 bis 13, das den Schritt des Vorhersagens des theoretischen Gehalts an Ipolamiid und seinen Derivaten der Formel C im Extrakt aus dem tatsächlichen Gehalt an Ipolamiid in der zugeführten pflanzlichen Biomasse unter Verwendung der Gleichung I enthält:

$$\text{\% Theoretischer Gehalt an Ipolamiid und Derivaten im Extrakt} = \frac{\text{\% Tatsächlicher Gehalt an Ipolamiid in der pflanzlichen Biomasse x DER}}{\dfrac{\text{(tatsächlicher Gehalt an Ipolamiid und Derivaten im Extrakt)}}{\text{(tatsächlicher Gehalt an Ipolamiid in der pflanzlichen Eingangsbiomasse)}}} \pm \text{Standardabweichung}$$

Gleichung I

**15.** Extrakte aus Pflanzen der Gattung *Stachytarpheta,* Ipolamiid-Derivaten der Formel C angereichert, wie sie in den Ansprüchen 1 oder 2 definiert ist.

**16.** Extrakte aus Pflanzen der Gattung *Stachytarpheta,* die mit Ipolamiid-Derivaten der Formel C angereichert sind, die nach dem Verfahren erhalten wurden, wie es in einem der Ansprüche 7 bis 14 definiert ist.

**17.** Extrakte aus Pflanzen der Gattung *Stachytarpheta* nach Anspruch 15 oder Anspruch 16, wobei 1 % bis 20 % Ipolamiid oder Ipolamiid-Derivate der Formel C enthaltend ist/sind.

**18.** Extrakte aus Pflanzen der Gattung *Stachytarpheta* nach einem der Ansprüche 15 bis 17 zur Verwendung als Immunsuppressiva bei der Behandlung immunologischer Erkrankungen.

## Revendications

**1.** Composés de formule générale C, qui sont des dérivés d’ipolamiide

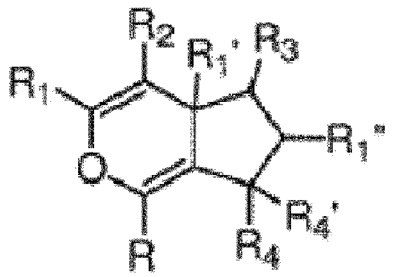

**C**

dans laquelle R correspond à H, OH, OGlyc (Glycoside) ;

$R_1$, $R_{1'}$, $R_{1''}$ correspondent à H, OH ;
$R_2$ correspond à H, COOH, $COOCH_3$, $CH_3$, CHO ;
$R_3$ correspond à H, OH, $CH_3$ ;
$R_4$, $R_{4'}$ correspondent à H, OH, $CH_2OH$, $CH_3$.

**2.** Composés de formule C selon la revendication 1, choisis parmi :

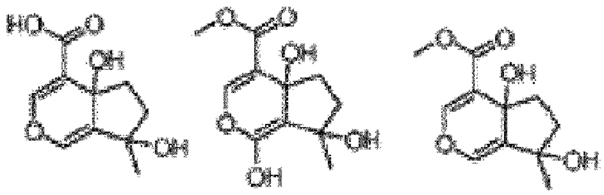

(IV) (V) (VIII)

**3.** Composés de formule C tels que définis dans l'une des revendications 1 ou 2, destinés à être utilisés comme médicaments.

**4.** Composés de formule C tels que définis dans l'une des revendications 1 ou 2, destinés à être utilisés comme immunosuppresseurs dans le traitement de troubles immunitaires.

**5.** Composés de formule C tels que définis dans l'une des revendications 1 ou 2, destinés à être utilisés dans le traitement du vitiligo.

**6.** Une composition pharmaceutique comprenant des dérivés d'ipolamiide de formule C tels que définis dans l'une des revendications 1 ou 2, et un véhicule pharmaceutiquement acceptable, comprenant éventuellement en outre de l'ipolamiide.

**7.** Procédé de production d'un extrait enrichi en dérivés d'ipolamiide de formule C tels que définis dans l'une des revendications 1 ou 2, comprenant les étapes suivantes :

a) sélectionner une biomasse végétale d'entrée ayant une teneur en ipolamiide comprise entre 2,5% et 3,5% obtenue à partir de plantes du genre *Stachytarphéta ;*
b) soumettre la biomasse sélectionnée en a) à un séchage au four à une température comprise entre 40 et 80°C, jusqu'à obtenir une stabilisation de l'humidité entre 10 et 12% ;
c) broyer la biomasse végétale ;
d) effectuer l'extraction de la biomasse végétale via les étapes suivantes:

i. chauffage de la biomasse végétale à une température comprise entre 70 et 100°C, avec une agitation constante ;
ii. macération de la biomasse végétale à température ambiante ;
iii. chauffage de la biomasse végétale à une température comprise entre 70 et 100°C.

**8.** Procédé de production d’un extrait selon la revendication 7, comprenant en outre les étapes suivantes:

iv. filtration et concentration de l’extrait ;
v. séchage dans un Séchoir à Pulvérisation, pendant de 1 à 60 secondes, avec une température d’entrée comprise entre 155 et 165°C et une température de sortie comprise entre 85 et 95°C, couplé à un déshumidificateur.

**9.** Procédé de production d’un extrait selon la revendication 7, dans lequel le chauffage de la biomasse végétale du d) point iii) s’effectue pendant 5 à 30 minutes.

**10.** Procédé de production d’un extrait selon la revendication 7, dans lequel le chauffage de la biomasse végétale du d) point i) s’effectue pendant 5 à 15 heures.

**11.** Procédé de production d’un extrait selon la revendication 7, dans lequel la biomasse végétale d’entrée comprend les parties aériennes des plantes du genre *Stachytarphéta.*

**12.** Procédé de production d’un extrait selon la revendication 7, dans lequel la plante est du *Stachytarphéta cayennensis.*

**13.** Procédé de production de l’extrait selon la revendication 7, comprenant une extraction aqueuse.

**14.** Procédé de production de l’extrait selon l’une quelconque des revendications 7 à 13, comprenant l’étape consistant à prédire la teneur théorique en ipolamiide et ses dérivés de formule C dans l’extrait à partir de la teneur réelle en ipolamiide dans la biomasse végétale d’entrée en utilisant l’Equation I :

*%Teneur théorique d'ipolamiide et de dérivés dans l'extrait* **=** *% Teneur réelle d'ipolamiide dans la biomasse végétale x DER*
*(contenu réel d'ipolamiide et de dérivés dans l'extrait)*
*(contenu réel d'ipolamiide dans la biomasse végétale d'entrée)* **±** *écart-type.*

## Équation I

**15.** Extraits de plantes du genre *Stachytarphéta* enrichis en dérivés d’ipolamiide de formule C tels que définis dans l’une des revendications 1 ou 2.

**16.** Extraits de plantes du genre *Stachytarphéta* enrichis en dérivés d’ipolamiide de formule C, obtenus par le procédé tel que défini dans l’une quelconque des revendications 7 à 14.

**17.** Extraits de plantes du genre *Stachytarphéta* selon l’une des revendications 15 et 16, contenant de 1% à 20% d’ipolamiide ou de dérivés d’ipolamiide de formule C.

**18.** Extraits de plantes du genre *Stachytarphéta* tels que définis dans l’une quelconque des revendications 15 à 17, pour une utilisation comme immunosuppresseurs dans le traitement des troubles immunologiques.

Figure 1

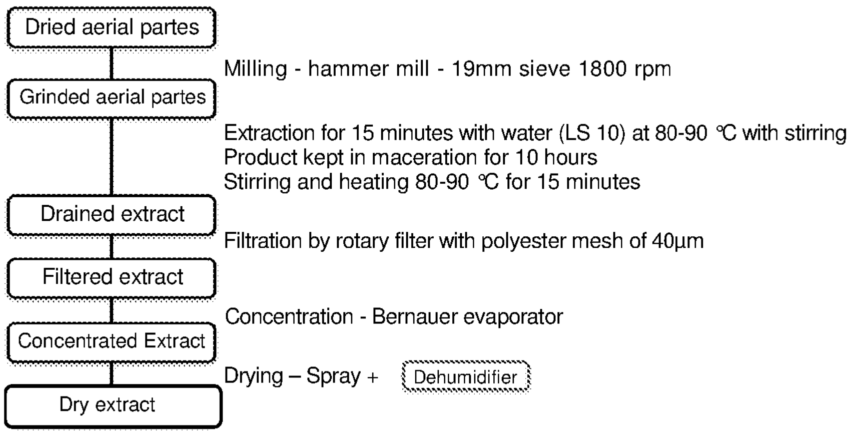
Dried aerial partes
Milling - hammer mill - 19mm sieve 1800 rpm
Grinded aerial partes
Extraction for 15 minutes with water (LS 10) at 80-90 °C with stirring
Product kept in maceration for 10 hours
Stirring and heating 80-90 °C for 15 minutes
Drained extract
Filtration by rotary filter with polyester mesh of 40µm
Filtered extract
Concentration - Bernauer evaporator
Concentrated Extract
Drying – Spray +
Dehumidifier
Dry extract

Figure 2

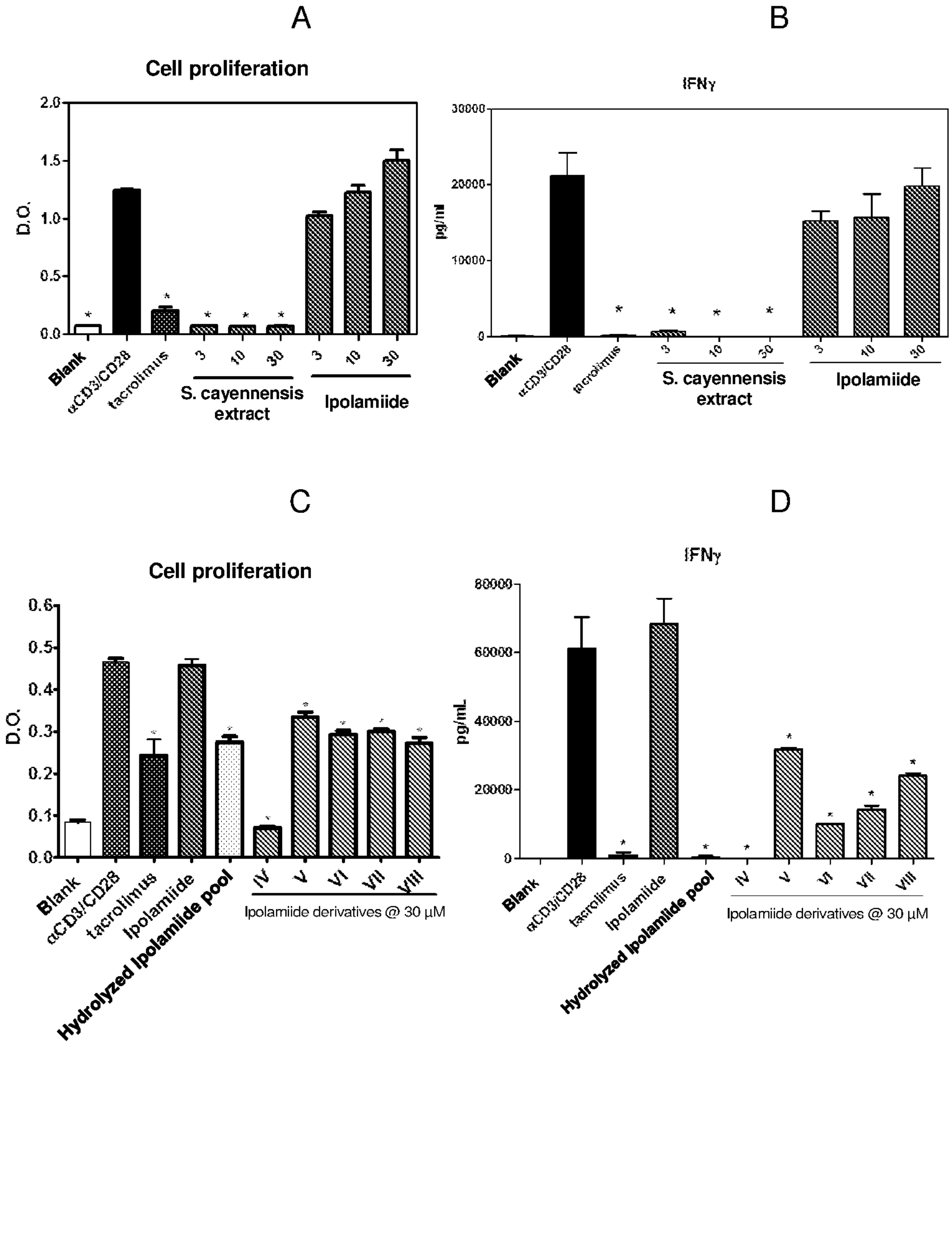
A
Cell proliferation
D.O.
2.0
1.5
1.0
0.5
0.0
Blank
αCD3/CD28
tacrolimus
3
10
30
S. cayennensis extract
Ipolamiide
B
IFNγ
pg/ml
30000
20000
10000
0
C
Cell proliferation
D.O.
0.6
0.5
0.4
0.3
0.2
0.1
0.0
Ipolamiide
Hydrolyzed Ipolamiide pool
IV
V
VI
VII
VIII
Ipolamiide derivatives @ 30 µM
D
IFNγ
pg/mL
80000
60000
40000
20000
0

Figure 3

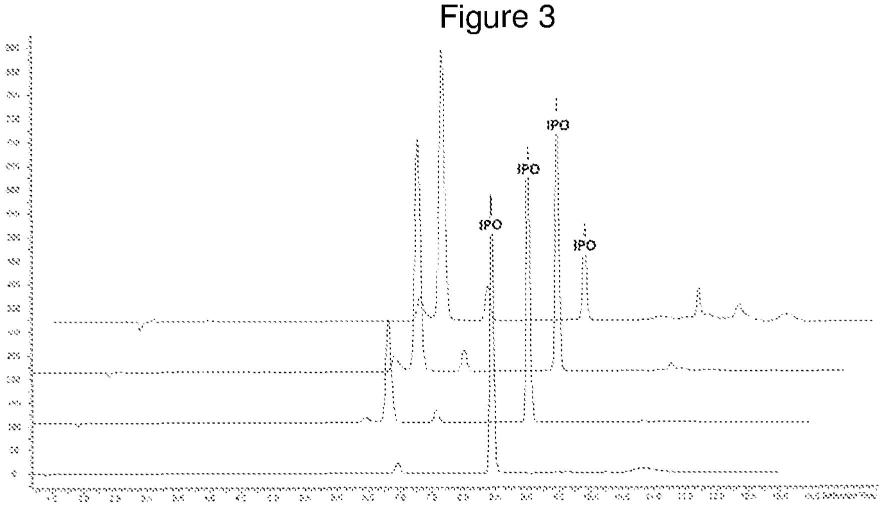

Figure 4

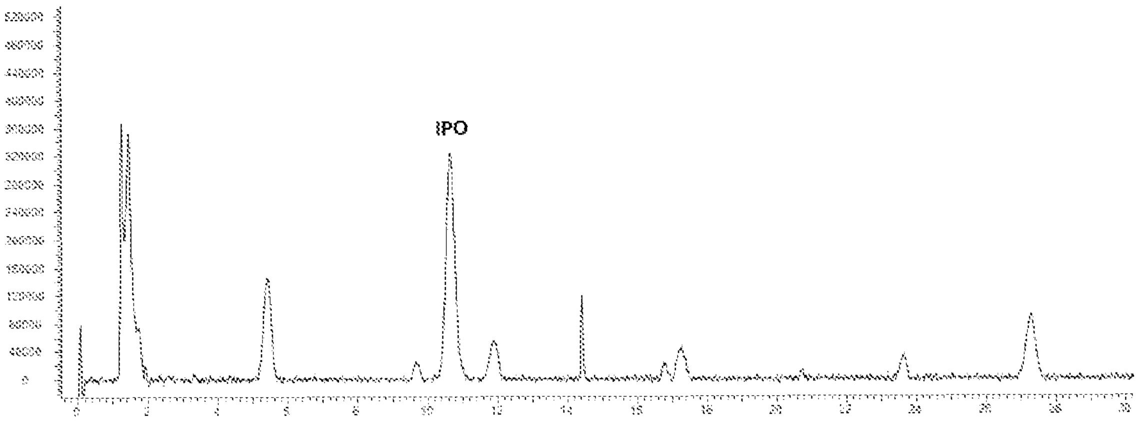

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016128471 A1
- WO 2008098325 A1
- WO 03094946 A1
- CN 103690551 A
- CN 103120699 A
- EP 1145709 A1
- EP 0045837 A1
- US 3703543 A
- DE 19644422 A1
- EP 1371372 A1

### Non-patent literature cited in the description


- **ADEBAJO A.C. et al.** *Planta Medica,* 2007, vol. 73 (3), 241-250
- **BIANCO A. et al.** *Tetrahedron Letters,* 1978, vol. 48, 4829-4832
- **KHOBRAKOVA V.B. et al.** *Pharmaceutical Chemistry Journal,* 2017, vol. 51 (5), 384-387
- **HOKI KUNIHITO et al.** *Journal of Physical Chemistry B,* 2004, vol. 108 (15), 4916-4921
- **KUNIHITO HOKI et al.** *Journal of Chemical Physics,* 2003, vol. 119 (23), 12393-12398
- **SCHLOEGL K. et al.** *Monatshefte fuer chemie,* 1964, vol. 95 (2), 558-575
- **BENKESER ROBERT A. et al.** *Journal of the American Chemical Society,* 1964, vol. 86 (5), 890-895
- **ORHAN ILKAY ERDOGAN et al.** *Industrial Crops and Products,* 2016, vol. 81, 117-121
- **KOBAYASHI YUKO et al.** *Bioscience, Biotechnology and Biochemistry,* 2016, vol. 80 (6), 1172-1178
- **LEE JE-HYUK et al.** *Food Science and Biotechnology,* 2009, vol. 18 (6), 1371-1378
- **LEE JE-HYUK et al.** *Journal of the Science of Food and Agriculture,* 2009, vol. 89 (10), 1762-1769
- **YU XIA et al.** *Journal of Chinese Pharmaceutical Sciences,* 2009, vol. 18 (3), 232-235
- **ELMASRI WAEL A et al.** *Rapid Communications in Mass Spectrometry,* 2016, vol. 30 (18), 2033-2042
- **BADEN CHRISTIAN ULRICH et al.** *Chemoecology,* 2012, vol. 22 (2), 113-118
- **AKKOL ESRA KUPELI et al.** *Pharmaceutical Biology,* 2009, vol. 47 (3), 188-194
- **VICCINI et al.** *Journal of Molecular Structure,* 2008, vol. 875 (1-3), 27-31
- **LEITAO GILDA GUIMARAES et al.** *Journal of Liquid Chromatography & Related Technologies,* 2005, vol. 28 (12-13), 2053-2060
- **SCHAPOVAL ELFRIDES E.S.** *Journal of Ethnopharmacology,* 1998, vol. 60 (1), 53-59
- **SARFARAZ K. NIAZI.** Handbook of Pharmaceutical Manufacturing Formulations. CRC Press, 2004, vol. 1,2,3,4,5,6
- Remington's Pharmaceutical Sciences. Mack Publishing